# EUROPEAN PATENT APPLICATION

(11) **EP 4 706 744 A1**
(43) Date of publication of application: **11.03.2026**
(21) Application number: 24198846.8
(22) Date of filing: 06.09.2024
(51) Int. Cl.: A61N 1/04, A61N 1/39

(54) **INTEGRATED ADULT AND CHILD DEFIBRILLATOR PADDLE**

(71) Applicant: Koninklijke Philips N.V., 5656 AG Eindhoven (NL)
(72) Inventor: YANG, Chao, Eindhoven (NL); WU, Jikun, Eindhoven (NL); ZHANG, Yuyang, 5656AG Eindhoven (NL); CHEN, Chaobin, Eindhoven (NL)
(74) Representative: Philips Intellectual Property & Standards

(57) **Abstract**

A defibrillator paddle comprising a first electrode plate and a second electrode plate. The second electrode plate may be moved relative to the first electrode plate to switch the defibrillator paddle between two different arrangements. In a first arrangement, the two electrode plates are aligned and may be used together as effectively a single large electrode plate. In a second arrangement, the second electrode plate is retracted relative to the first electrode plate, such that the first electrode plate may be used on its own. Accordingly, the first and second arrangements may correspond to adult and child modes of the defibrillator paddle, respectively. Additionally, in both arrangements, the first and second electrode plates are exposed.

## Description

### FIELD OF THE INVENTION

The present invention relates to the field of defibrillators, and in particular to defibrillator paddles comprising adult and child electrode plates.

### BACKGROUND OF THE INVENTION

Defibrillators are commonly used to treat patients suffering from cardiac arrest and/or heart arrythmia. Electrode plates arranged on paddles of the defibrillator are pressed against the patient's body to provide an electric shock to the patient's heart through a short burst of electrical current.

Safe and reliable defibrillation of child patients usually requires electrode plates of a smaller lateral size compared to those used on adult patients, e.g., to ensure full contact between the electrode plate surface and the child's body. Certain defibrillator paddles may comprise both child electrode plates and adult electrode plates to allow them to be used in both scenarios. In some examples, the child electrode plate is positioned under (i.e., covered by) the adult electrode plate, and can be exposed by (completely) removing the adult electrode plate.

This configuration can, however, result in increased wear to the child electrode plate due to the repeated removal and replacement of the adult electrode plate. Additionally, conductive gel (commonly used in defibrillation to enhance the electrical conductivity between the electrode plates and the patient) may become trapped between the adult and child electrode plates if not cleaned off properly, which can cause degradation (e.g., rusting) of both electrode plates over time.

### SUMMARY OF THE INVENTION

The invention is defined by the claims.

According to examples in accordance with an aspect of the invention, there is provided a defibrillator paddle comprising a first electrode plate comprising a first shock surface, and a second electrode plate comprising a second shock surface. The second electrode plate is configured to be moveable with respect to the first electrode plate.

In a first arrangement of the defibrillator paddle, the first shock surface and the second shock surface both lie in a first plane and face a same direction. In a second arrangement of the defibrillator paddle, the first shock surface lies in the first plane and the second shock surface lies in a second plane different to the first plane. In the first arrangement and the second arrangement of the defibrillator paddle, the first shock surface and the second shock surface are exposed.

The present disclosure provides a defibrillator paddle (e.g., for use as part of a defibrillator) comprising a first electrode plate and a second electrode plate. Each electrode plate comprises a shock surface being the (intended) side/surface of the electrode plate that comes in contact with a subject when delivering an electric shock. Additionally, the second electrode plate is moveable with respect to the first electrode plate. Said movement may correspond to adjustment/changing of a position of the second electrode plate on the defibrillator paddle, and/or removal of the second electrode plate from the defibrillator paddle entirely.

The defibrillator paddle is configured to operate in (at least) two modes/configurations corresponding to relative arrangements of the first and second electrode plates. In a first arrangement, the first and second electrode plates are aligned such that their shock surfaces lie in a shared (i.e., first) plane and are facing the same direction. In this way, the first and second electrode plates may be used together as a single (i.e., combined/integrated) electrode plate during defibrillation, e.g., for use on an adult. In other words, in the first arrangement, the overall shock surface for the defibrillator paddle is made from the combination of the first and second electrode plates.

In a second arrangement, a position of the second electrode plate is modified such that its shock surface lies in a plane different to that for the shock surface of the first electrode plate (i.e., a second plane). Particularly, in such an arrangement, the first electrode plate may be used on its own during defibrillation, e.g., for use on a child/infant. In other words, in the second arrangement, only the first electrode plate contributes towards the overall shock surface for the defibrillator paddle.

By switching between the first and second arrangements, the (effective) size of the shock surface for the defibrillator paddle may be adjusted for the appropriate use on subjects of different size and age.

Additionally, in both the first and second arrangements, the shock surfaces for both the first and second electrode are exposed. In this way, wear and/or degradation of the electrode plates is reduced (compared to the prior art), e.g., due to the reduced likelihood of one electrode plate rubbing against another, and/or prevention/reduction of trapped conductive gel.

Typically, the defibrillator paddle may be configured to receive electrical power from an electrical shock system. In such cases, said electrical power may be provided to at least the first electrode plate.

Additionally, in the first arrangement of the defibrillator paddle, the second electrode plate may be configured to come into electrical contact with the first electrode plate. Put another way, the second electrode plate may be configured to, in the first arrangement, receive electrical power from the electrical shock system via the first electrode plate. Thus, in the first arrangement, both the first and second electrode plates may be electrically active, e.g., for the purposes of providing an electric shock to a subject.

In some examples, the electrical contact (between the first and second electrode plates) may be made via a plurality of resilient protrusions arranged on the second electrode plate. Specifically, in the first arrangement, the plurality of resilient protrusions are configured to come into contact with the first electrode plate. In this way, a good/strong and reliable electrical connection may be made between the second electrode plate and the first electrode plate.

Additionally, or alternatively, in the second arrangement, the second electrode plate may be electrically isolated from the first electrode plate. Thus, in the second arrangement, the second electrode plate may not be electrically active.

In some examples, the second plane may be parallel to the first plane.

The defibrillator paddle may further comprise an electrode plate holder coupled to the first electrode plate and the second electrode plate. Particularly, the first electrode plate may be rigidly connected to the electrode plate holder, and the second electrode plate may be configured to be moveable along an axis of the electrode plate holder. Furthermore, said axis may make a non-zero angle with respect to the first plane and the second plane.

In particular, the defibrillator paddle may be switched between the first and second arrangement by moving (e.g., sliding/translating) the second electrode plate along the axis of the electrode plate holder.

In some examples, the axis of the electrode plate holder may be perpendicular to the first plane and the second plane.

In some examples, the first arrangement and the second arrangement of the defibrillator paddle may correspond to extreme positions of the second electrode plate along the axis of the electrode plate holder.

For example, the first arrangement may correspond to the second electrode plate being positioned at one end of the electrode plate holder, while the second arrangement may correspond to the second electrode plate being positioned at another (i.e., opposite) end of the electrode plate holder.

In some examples, the second electrode plate may comprise a biased plunger configured to, when biased, prevent movement of the second electrode plate along the axis of the electrode plate holder at least when the defibrillator paddle is in the first arrangement and the second arrangement.

Said biasing may be induced, e.g., by a spring or other resilient object. Additionally, said biasing may be (temporarily) removed by a user, e.g., through applying a (manual) force to the biased plunger in the opposite direction of the biasing.

Furthermore, the electrode plate holder may comprise a first locking hole configured to receive the biased plunger in the first arrangement, and a second locking hole configured to receive the biased plunger in the second arrangement.

In particular, when receiving the biased plunger, the first and second locking holes may prevent movement of the second electrode plate along the axis of the electrode plate holder.

The electrode plate holder may further comprise a first microswitch and a second microswitch. Particularly, when received by the first locking hole, the biased plunger may act on the first microswitch, and when received by the second locking hole, the biased plunger may act on the second microswitch. Accordingly, when acted on by the biased plunger the first microswitch may be configured to provide an indication of the defibrillator paddle being in the first arrangement, and the second microswitch may be configured to provide an indication of the defibrillator paddle being in the second arrangement.

In some examples, the second electrode plate may comprise an aperture having bounds that surround the first electrode plate when the defibrillator paddle is in the first arrangement.

In some examples, an area of the second shock surface may be greater than or equal to an area of the first shock surface.

In some examples, the second electrode plate may be configured to move responsive to an applied force from a user.

Also provided is a defibrillator comprising an electrical shock system, a processing system configured to control the electrical shock system, and at least one defibrillator paddle according to the above disclosure. Particularly, the at least one defibrillator paddle is configured to receive electrical power from the electrical shock system.

In some examples, the processing system may be configured to control at least an output energy of the electrical shock system responsive to an indication of the at least one defibrillator paddle being in either the first arrangement or the second arrangement.

Put another way, the defibrillator may be configured to switch between different modes of operation (e.g., adult mode and child mode) responsive to a change in the arrangement of the defibrillator paddle.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates an example of a defibrillator paddle;
Fig. 2 illustrates a proposed defibrillator paddle;
Fig. 3 illustrates an exploded view of a portion of the defibrillator paddle;
Fig. 4 illustrates another view of a portion of the defibrillator paddle; and
Fig. 5 illustrates a proposed defibrillator.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a defibrillator paddle comprising a first electrode plate and a second electrode plate. Each electrode plate comprises a respective shock surface, i.e., first and second shock surfaces. The defibrillator paddle is configured to operate in (at least) two different arrangements corresponding to relative arrangements of the first and second electrode plates. More precisely, the two arrangements correspond to relative alignments of the shock surfaces of the two electrode plates. Specifically, in a first arrangement (of the defibrillator paddle) the first and second shock surfaces both lie in a first plane and face a same direction, whereas, in a second arrangement, the first and second shock surfaces lie in different planes, i.e., first and second planes, respectively. Furthermore, in both the first and second arrangements, the first and second shock surfaces are exposed, i.e., not covered by another part of the defibrillator paddle.

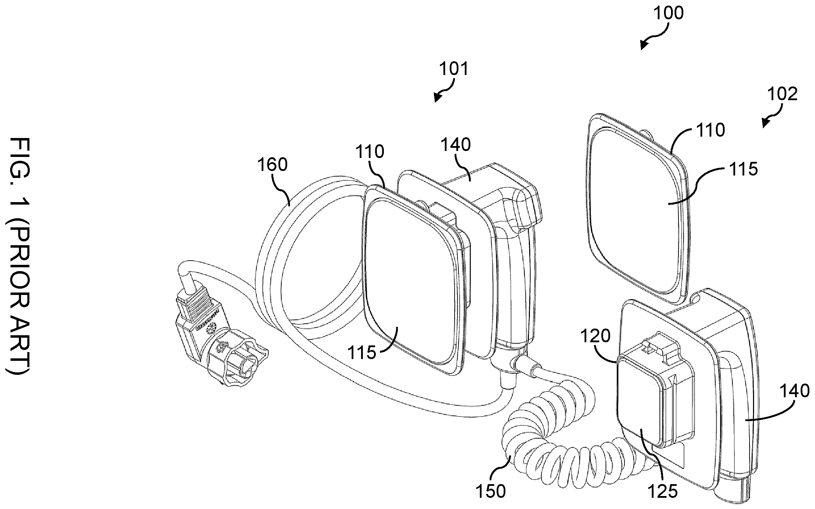

Fig. 1 shows an example of a defibrillator paddle system 100 comprising a pair of defibrillator paddles 101, 102 as known from the prior art. The pair of defibrillator paddles are (electrically) coupled via a first flexible cable 150, i.e., each defibrillator paddle 101, 102 is connected to the first flexible cable 150. One defibrillator paddle 101, 102 is further connected to a second flexible cable 160.

Fig. 1 thereby corresponds to a standard configuration for defibrillator paddles of a defibrillator as known to those skilled in the art. Specifically, the second flexible cable 160 may be connected to an electrical shock system of a defibrillator for the provision of electrical power to the pair of defibrillator paddles 101, 102, e.g., during defibrillation of a subject/patient. The first flexible cable 150 and second flexible cable 160 may further allow for increased maneuverability of the pair of defibrillator paddles, e.g., to allow the defibrillator paddles to be easily positioned on desired regions of a subject.

Each defibrillator paddle 101, 102 comprises an adult electrode plate 110 and a child electrode plate 120. Particularly, the adult and child electrode plates correspond to desired electrode plates to be used when defibrillating subjects of a particular age and/or size/weight. More particularly, the adult electrode plate 110 may be used predominantly, i.e., designed for use, on adult subjects, whereas the child electrode plate 120 may be used predominantly, i.e., designed for use, on child/infant subjects.

More precisely, the adult electrode plate 110 or the child electrode plate 120 may be used dependent on whether a subject's age and/or size breaches one or more predetermined thresholds. For example, the adult electrode plate 110 may be preferably used on subjects older than 8 years old and/or over 25 kg. Conversely, the child electrode plate 120 may be preferably used on subjects younger than 8 years old and/or under 25 kg. The selection of which electrode plate to use will be readily apparent to the skilled person.

Accordingly, an active electrode plate of the/each defibrillator paddle 101, 102, i.e., the electrode plate being or to be used for defibrillation, may be chosen dependent on the intended subject to be defibrillated. Furthermore, the active electrode plate of each defibrillator paddle may be set dependent on an arrangement/configuration of the adult and child electrode plates on the defibrillator paddle 101, 102, as will be later disclosed.

Each electrode plate comprises a shock surface 115, 125 being the intended side/surface of the electrode plate to be in contact with the subject during defibrillation. Particularly, during defibrillation, electrical energy is transferred from the defibrillator paddle 101, 102 to the subject via the shock surface of the electrode plate being used.

An adult shock surface 115 of the adult electrode plate 110 has a larger area than a child shock surface 125 of the child electrode plate 120, reflective of the size of the subject that each electrode plate is intended to be used on. To provide more context, during defibrillation, it is desirable for the entirety of the shock surface to be in contact with the subject, e.g., for increased safety and defibrillation effectiveness. Accordingly, the child shock surface 125 has a smaller area (compared to the adult shock surface 115) to align with the reduced size of the intended subject, e.g., a child or infant.

Each defibrillator paddle 101, 102 further comprises a handle 140 to be held by a user when operating the defibrillator paddle 101, 102.

Each defibrillator paddle 101, 102 is configured to operate (at any one moment) in one of two arrangements, each corresponding to a relative arrangement of the adult and child electrode plates. Particularly, in Fig. 1, each defibrillator paddle 101, 102 is depicted as being in a respective one of the two arrangements.

A first defibrillator paddle 101 is depicted in an adult mode arrangement 101, the adult electrode plate 110 is positioned on top of (i.e., attached to) the child electrode plate 120, such that the child shock surface 125 is entirely covered by the adult electrode plate 110. In this arrangement, the adult electrode plate 110 may be used as the active electrode plate of the first defibrillator paddle 101. Accordingly, the defibrillator paddle 101 may be operated in the adult mode arrangement, e.g., for use when defibrillating an adult subject and/or a subject whose age and/or size breach one or more predetermined thresholds.

A second defibrillator paddle 102 is depicted in a child mode arrangement, the adult electrode plate 110 is detached from the second defibrillator paddle 102, such that the child shock surface 125 is entirely exposed. In this arrangement, the child electrode plate 120 may be used as the active electrode plate of the second defibrillator paddle 102. Accordingly, the second defibrillator paddle 102 may be operated in the child mode arrangement 102, e.g., for use when defibrillating a child subject and/or a subject whose age and/or size are below one or more predetermined thresholds.

It will be appreciated that the first and second defibrillator paddles are switchable between the adult mode arrangement (illustrated with reference to the first defibrillator paddle) and the child mode arrangement (illustrated with reference to the second defibrillator paddle) by way of controllably removing and attaching the adult electrode plate to the defibrillator paddle.

To provide further context to the disclosed arrangements, typically, the child electrode plate 120 receives (or can receive) electrical power (e.g., from an electrical shock system) in both the child mode arrangement (as illustrated with reference to the second electrode paddle 102) and the adult mode arrangement (as illustrated with reference to the first electrode paddle 102). More precisely, electrical power received by the defibrillator paddle 101, 102 is provided to the child electrode plate 120 in both arrangements. Furthermore, in the adult mode arrangement, the adult electrode plate 110 is configured to be in electrical contact with the child electrode plate 120. Accordingly, when the defibrillator paddle 101, 102 is powered in the adult mode arrangement, the adult electrode plate 110 receives electrical power via the child electrode plate 120.

As will be evident from the above disclosure, the defibrillator paddle 101, 102 can be switched between the adult mode and child mode arrangements through detachment (and reattachment) of the adult electrode plate 110. In other words, the adult electrode plate 110 is configured to be detachable from the defibrillator paddle 101, 102 (or more specifically from the child electrode plate 120). Conversely, the child electrode plate 120 is rigidly coupled to the defibrillator paddle 101, 102, i.e., non-detachable.

The present disclosure recognizes that the present configuration for the adult and child electrode plates on the defibrillator paddle 101, 102 may have a number of disadvantages. Firstly, switching to the child mode arrangement 102 necessitates the complete removal of the adult electrode plate 110. This naturally brings with it the risk of losing or otherwise misplacing the adult electrode plate 110, particularly when considering the high-stress environments/circumstances in which the defibrillator paddle 101, 102 may be used, e.g., a subject suffering from cardiac arrest.

Additionally, repeated detachment and reattachment of the adult electrode plate 110 from/to the child electrode plate 120 may result in increased wear of the child electrode plate 120, or more precisely of the child shock surface 125. Particularly, each time the adult electrode plate 110 is removed or reattached it may rub against the child shock surface 125, potentially resulting in scratching and/or other physical damage to the child shock surface 125 which can impede its electrical/defibrillation performance.

As another example, during defibrillation, a conductive gel is typically applied between the shock surfaces of the defibrillator paddles and the subject's skin to improve the transfer of electrical energy. This gel should preferably be cleaned off after use to prevent degradation (e.g., rusting) of the electrode plates. Consequently, if the child electrode plate 120 is not properly cleaned after use and the adult electrode plate 110 is reattached, the conductive gel can become trapped between the adult and child electrode plates. This can cause gradual degradation to the child shock surface 125 and/or internal components/circuitry of the adult electrode plate 110, potentially affecting the performance of both electrode plates.

The present disclosure proposes a mechanism for avoiding or mitigating at least some of the above disadvantages that have been herein identified.

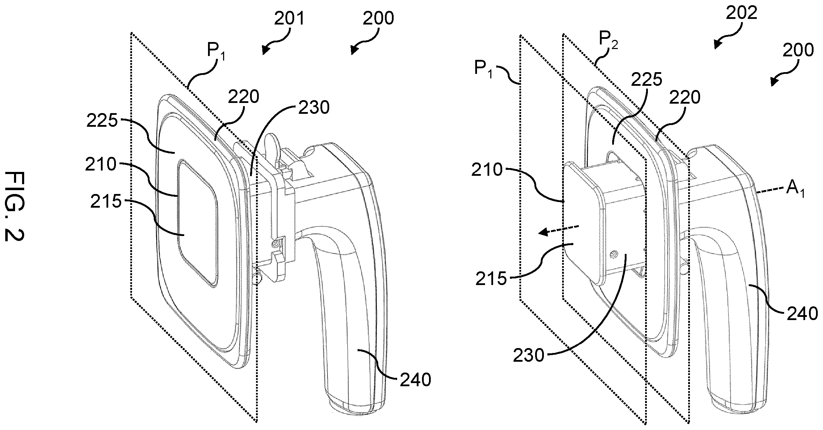

Fig. 2 shows a proposed defibrillator paddle 200. Particularly, the defibrillator paddle 200 is depicted in a first arrangement 201 and a second arrangement 202 corresponding to possible arrangements and/or modes of operation of the defibrillator paddle 200 for defibrillating different subjects, as will be later disclosed.

For conciseness, the first arrangement 201 of the defibrillator paddle 200 and the second arrangement 202 of the defibrillator paddle 200 will be simply referred to as the first arrangement 201 and the second arrangement 202, respectively.

The defibrillator paddle 200 comprises a first electrode plate 210 having a first shock surface 215, and a second electrode plate 220 having a second shock surface 225. The shock surface for a respective electrode plate corresponds to an intended side/surface of the electrode plate to be in contact with a subject during defibrillation (i.e., when said electrode plate is used).

The second electrode plate 220 is configured to be moveable with respect to the first electrode plate 210. For example, this may correspond to the first electrode plate 210 having a fixed position on the defibrillator paddle 200 and the second electrode plate 220 being moveable with respect to the remainder of the defibrillator paddle 200. Furthermore, said movement of the second electrode plate 220 may correspond to an adjustment of a position of the second electrode plate 220 on the defibrillator paddle 200 and/or to a removal (i.e., detachment) of the second electrode plate 220 from the defibrillator paddle 200.

The defibrillator paddle 200 may be switched between the first arrangement 201 and the second arrangement 202 through movement of the second electrode plate 220. More particularly, the first and second arrangements of the defibrillator paddle 200 may be defined from the relative arrangement and/or alignment of the first electrode plate 210 and the second electrode plate 220.

In the first arrangement 201, the first and second electrode plates are substantially aligned such that the first shock surface 215 and the second shock surface 225 both lie in a first plane P₁. Additionally, the first shock surface 215 and the second shock surface 225 both face a same direction.

Preferably, the first electrode plate 210 and the second electrode plate 220 are substantially close/proximate to each other. In this way, the first shock surface 215 and the second shock surface 225 may form an approximately continuous single surface. Put another way, in the first arrangement 201, the first shock surface 215 and the second shock surface 225 may be combined to form a single larger shock surface.

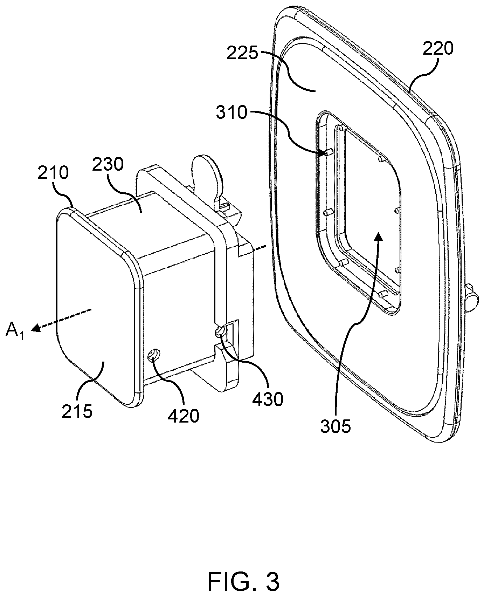

In the illustrated example, the second electrode plate 220 may comprise an aperture, i.e., a hole. This can be seen more clearly in Fig. 3, which provides an exploded view of a portion of the defibrillator paddle. Specifically, said aperture 305 may have a lateral size and shape substantially similar to that of the first electrode plate 210. In this way, in the first arrangement 201, the bounds of the aperture may surround the first electrode plate 210, such that the first shock surface 215 and the second shock surface 225 may form an approximately continuous single surface.

Of course, in certain embodiments, the second electrode plate 220 may not comprise an aperture. In such cases, in the first arrangement 201, the first and second electrode plates may be positioned substantially adjacent to each other, such that the first shock surface 215 and the second shock surface 225 may form an approximately continuous single surface.

In accordance with the above disclosure, the first arrangement 201 may correspond to a mode of operation of the defibrillator paddle 200 in which both the first 210 and second 320 electrode plates are used during defibrillation. In particular, the first electrode plate 210 and the second electrode plate 220 may be used in combination to (effectively) form a single larger electrode plate. Accordingly, the first arrangement 201 may be suitable for defibrillation of adult subjects and/or subjects whose age and/or size breach one or more predetermined thresholds, e.g., older than 8 years old and/or over 25 kg.

In the second arrangement 202, the second electrode plate 220 is moved (compared to the first arrangement 201) such that the second shock surface 225 lies in a second plane P₂ different to the first plane P₁. Meanwhile, the first shock surface 215 still lies in the first plane P1. In other words, the position of the first electrode plate 210 on the defibrillator paddle 200 is not changed between the first arrangement 201 and the second arrangement 202.

According to the above disclosure, the first plane P₁ may be considered as (or otherwise defined as) any plane occupied by the first shock surface 215.

On changing from the first arrangement 201 to the second arrangement 202, the second electrode plate 220 is moved with respect to the first electrode plate 210. For example, as depicted in Fig. 2, the second electrode plate 220 may be translated along a predefined direction (and/or by a predefined distance) to move the second shock surface 225 into the second plane P₂. Accordingly, in such examples, the second plane P₂ may be (substantially) parallel to the first plane P₁.

In further examples, the second electrode plate may be rotated around one or more predefined axes to move the second shock surface 225 into the second plane P₂, or moved through a combination of one or more rotations and/or translations. In such examples, the second electrode plate 220 may remain attached to the defibrillator paddle 200 throughout the predefined movement. Accordingly, the second plane P₂ may be a predefined plane as set by the predefined movement.

Alternatively, changing from the first arrangement 201 to the second arrangement 202 may comprise detaching the second electrode plate 220 from the defibrillator paddle 200. Accordingly, the second plane P₂ may be any arbitrary plane different to the first plane P₁. Put another way, the second plane P₂ may simply be any plane occupied by the second shock surface 225 when the second electrode plate 220 is detached from the defibrillator paddle 200.

Preferably, in the second arrangement 202, a region in front of the first shock surface 215 is unobstructed by the second electrode plate 220 (or any other component of the defibrillator paddle 200). For example, as depicted in Fig. 2, in the second arrangement 202, the second electrode plate 220 may be in a retracted position/configuration, i.e., positioned behind the first shock surface 215. In this way, during defibrillation, contact may be made between a subject and the first shock surface 215 and no other part of the defibrillator paddle 200, e.g., the second shock surface 225.

In accordance with the above disclosure, the second arrangement 202 may correspond to a mode of operation of the defibrillator paddle 200 in which only the first electrode plate 210 is used during defibrillation. In particular, in the second arrangement 202, the second electrode plate 220 may be inactive and/or not make contact with a subject during defibrillation. Accordingly, the second arrangement 202 may be suitable for defibrillation of child subjects and/or subjects whose age and/or size are below one or more predetermined thresholds, e.g., younger than 8 years old and/or below 25 kg.

Typically, the first shock surface 215 may have an area less than (or at most equal to) an area of the second shock surface 225. In this way, an active shock surface of the defibrillator paddle 200 in the second arrangement 202 (i.e., the first shock surface 215) may have an area no greater than half of the area for the active shock surface of the defibrillator paddle 200 in the first arrangement 201 (i.e., the combination of the first shock surface 215 and the second shock surface 225).

Preferably, however, the area of the first shock surface 215 may be much less than half of the combined area of the first shock surface 215 and the second shock surface 225. For example, the first shock surface 215 may preferably have an area in the range 20-30 cm² and/or lateral dimensions (e.g., length and width) in the range 4.5-5.5 cm. Such a size may be suitable for use on child subjects. Additionally, the combination of the first shock surface 215 and the second shock surface 225 may preferably have an area in the range 90-130 cm² and/or lateral dimensions in the range 9.5-11.5 cm. Such a size may be suitable for use on adult subjects. Accordingly, the combination of the first and second shock surfaces may preferably have an area of from 3 to 6 times that of an area of the first shock surface alone.

In the first arrangement 201 and the second arrangement 202, both the first shock surface 215 and the second shock surface 225 are exposed, i.e., not covered by another part of the defibrillator paddle 200. Particularly, this may reduce the chance of conductive gel becoming trapped between electrode plates of the defibrillator paddle 200. Furthermore, said exposure may be maintained when switching between the first arrangement 201 and the second arrangement 202, i.e., one of the electrode plates does not (necessarily) need to cover and/or come into contact with the other when switching arrangements. This may greatly reduce the risk of scratching and/or wearing the shock surface(s) of one or more of the electrode plates, thereby increasing the lifetime of the defibrillator paddle 200.

According to a preferred embodiment, the defibrillator paddle 200 may comprise an electrode plate holder 230 that (physically) couples to the first electrode plate 210 and the second electrode plate 220. Particularly, the first electrode plate 210 may be rigidly connected to the electrode plate holder 230, i.e., fixed and/or immovable with respect to the electrode plate holder 230. Conversely, the second electrode plate 220 may be moveable with respect to the electrode plate holder 230.

In some examples, the second electrode plate 220 may be configured to be detachable from the electrode plate holder 230. In other words, the second electrode plate 220 may decouple from the electrode plate holder 230.

Preferably, the second electrode plate 220 may be configured to be moveable along an axis A₁ of the electrode plate holder 230. More specifically, when coupled to the electrode plate holder 230, the second electrode plate 220 may be configured to move (e.g., slide/translate) relative to the electrode plate holder 230 along the axis A₁. In such examples, the second shock surface 225 may be moved between the first plane P₁ and the second plane P₂ through movement of the second electrode plate 220 along the axis A₁. In other words, the defibrillator paddle 200 may be switched between the first arrangement 201 and the second arrangement 202 through movement of the second electrode plate 220 along the axis A₁.

As depicted in Fig. 2, the axis A₁ may be perpendicular to the first plane P₁ and the second plane P₂, i.e., form a 90° angle with the first plane P₁ and the second plane P₂. Accordingly, the axis A₁ may also be perpendicular to the first shock surface 215 and the second shock surface 225.

Of course, however, the axis A₁ may make any non-zero angle (e.g., 30°, 45°, 60°, etc.) with respect to the first plane P₁ and the second plane P₂. Accordingly, movement of the second electrode plate 220 along the axis A1 may move the second shock surface 225 in and out of alignment with the first plane P₁.

As further depicted in Fig. 2, in embodiments where the second electrode plate 220 comprises an aperture, the bounds of said aperture may surround the electrode plate holder 230. In particular, the second electrode plate 220 may be coupled to the electrode plate holder 230 via the aperture, where said coupling permits the second electrode plate 220 to move relative to the electrode plate holder 230 along the axis A₁.

Of course, in embodiments where the second electrode plate 220 does not comprise an aperture, the second electrode plate 220 may be coupled to the electrode plate holder 230 via a different part of the second electrode plate 220, e.g., an edge/periphery of the second electrode plate 220.

In some examples, the first arrangement 201 and the second arrangement 202 may correspond to extreme positions of the second electrode plate 202 along the axis A₁. In other words, the first arrangement 201 and the second arrangement 202 may correspond to limits in the allowed movement of the second electrode plate 220 along the axis A₁. Particularly, said limits may be defined by the electrode plate holder 230.

For example, the first arrangement 201 may correspond to the second electrode plate 220 being positioned as far as possible (e.g., as permitted by the electrode plate holder 230) in a first direction along the axis A₁. Additionally, the second arrangement 202 may correspond to the second electrode plate 220 being positioned as far as possible in a second direction opposite the first direction along the axis A₁.

The defibrillator paddle 200 may further comprise a handle 240 to be held by a user when operating the defibrillator paddle 200.

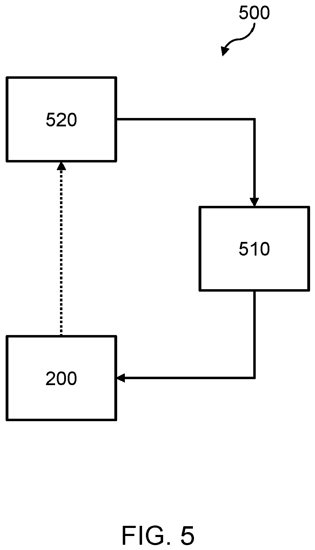

Typically, the defibrillator paddle 200 may be configured to receive electrical power (e.g., for the purposes of defibrillation) from an electrical shock system (e.g., the electrical shock system 510 of Fig. 5). More specifically, the defibrillator paddle 200 may comprise a wire/cable (not visible in Fig. 2) configured to form an electrical connection between the defibrillator paddle 200 and the electrical shock system. Accordingly, during defibrillation, the defibrillator paddle 200 may be provided electrical power from the electrical shock system.

The electrical power provided to the defibrillator paddle 200 may be provided to at least the first electrode plate 210. To be more precise, the electrical connection between the defibrillator paddle 200 and the electrical shock system may be (or may otherwise facilitate) an electrical connection between the first electrode plate 210 and the electrical shock system. Electrical power provided to the defibrillator paddle 200 may therefore be provided directly to the first electrode plate 210. Accordingly, in the second arrangement 202, the first electrode plate 210 may be used for the provision of an electric shock to a subject, i.e., through transfer of electrical energy (provided by the electrical shock system) from the first electrode plate 210 to the subject via the first shock surface 215.

Preferably, in the second arrangement 202, the second electrode plate 220 may be configured to not receive electrical power. In this way, the risk of a subject (and/or a user) receiving an electric shock from the second electrode plate 220 when the defibrillator paddle 200 is used in the second arrangement 202 is reduced.

For example, the defibrillator paddle 200 may be configured such that, in the second arrangement 202, the second electrode plate 220 is configured to be electrically isolated from the electrical shock system. In this way, only the first electrode plate 210 may be electrically active when the defibrillator paddle 200 is in the second arrangement 202.

In some cases, the second electrode plate 220 may not form a direct electrical connection with the electrical shock system. Rather, the second electrode plate 220 may receive electrical power from the electrical shock system via other components of the defibrillator paddle 200. For example, in accordance with a preferred embodiment, the second electrode plate 220 may be configured to (in certain arrangements) form an electrical connection with the first electrode plate 210. Thus, the electrical power provided to the first electrode plate 210 by the electrical shock system may be transferred to the second electrode plate 220.

More precisely, the second electrode plate 220 may only come into electrical contact with the first electrode plate 210 when the defibrillator paddle 200 is in the first arrangement 201. For example, in the first arrangement 201, a portion of the second electrode plate 220 may be in physical contact with a portion of the first electrode plate 210, where the contact between said portions provides an electrical connection between the first and second electrode plates. Thus, in the first arrangement 201, the second electrode plate 220 may be electrically active. In other words, both the first electrode plate 210 and the second electrode plate 220 may be used for the provision of an electric shock to a subject.

Conversely, the second electrode plate 220 may be electrically isolated from the first electrode plate 210 when the defibrillator paddle 200 is in the second arrangement 202. In other words, in the second arrangement 202, the second electrode plate 220 may not form an electrical connection with the first electrode plate 210. Accordingly, in the second arrangement 202, the second electrode plate 220 may not receive electrical power via the first electrode plate 210 and may thus be electrically inactive.

In some examples, in the first arrangement 201, the electrical connection between the first and second electrode plates may be made via a plurality of resilient protrusions 310 arranged on the second electrode plate 220, as shown in Fig. 3. More specifically, in the first arrangement 201, the plurality of resilient protrusions 310 (which are themselves conductive) may come into contact with the first electrode plate 210, thus forming an electrical connection.

Each resilient protrusion may comprise a biased conductive element (e.g., a metal pin coupled to a spring) and/or be made of a compressive material. In this way, in the first arrangement 201, the plurality of resilient protrusions 310 may be biased against the first electrode plate 210, thus forming a reliable electrical connection.

According to the present embodiment, the plurality of resilient protrusions 310 may be arranged in a recess region around the bounds of the aperture of the second electrode plate 220. Thus, in the first arrangement 201, the plurality of resilient protrusions 310 may come into contact with a back surface of the first electrode plate 210, i.e., a surface of the first electrode plate 210 opposite the first shock surface 215.

In examples where the second electrode plate 220 does not comprise an aperture, the plurality of resilient protrusions 310 may be arranged on an edge region (e.g., periphery) of the second electrode plate 220. More precisely, the plurality of resilient protrusions 310 may be arranged on an edge region that is closest to the first electrode plate 210 when the defibrillator paddle 200 is in the first arrangement 201.

As an alternative example, in the first arrangement 201, the second electrode plate 220 may not form an electrical connection with the first electrode plate 210. Instead, the second electrode plate 220 may form a different electrical connection with the electrical shock system, e.g., a direct connection, or via another component of the defibrillator paddle 200. Furthermore, in the second arrangement 202, said electrical connection between the second electrode plate 220 and the electrical shock system may be broken, such that the second electrode plate 220 may not receive electrical power.

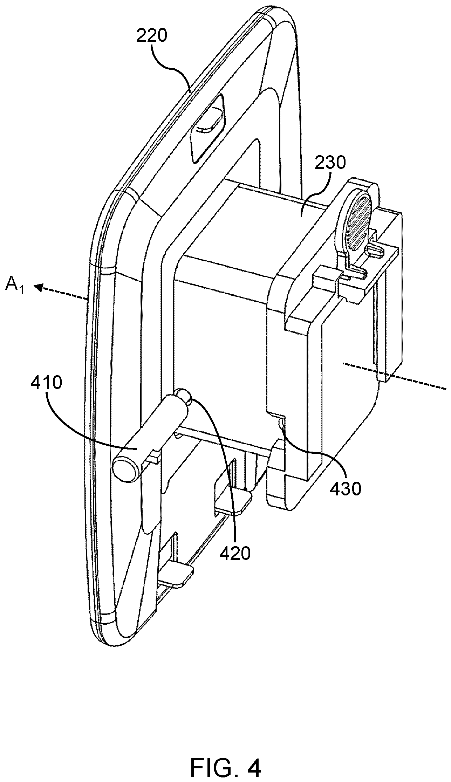

Fig. 4 shows a portion of the defibrillator paddle 200 comprising the first electrode plate 210 (not visible in Fig. 4), the second electrode plate 220, and the electrode plate holder 230.

Though the second electrode plate 220 is configured to be moveable with respect to the first electrode plate 210, it may be preferable for said movement to be prevented (or otherwise reduced or resisted) when the defibrillator paddle 200 is in the first arrangement 201 and/or the second arrangement 202. Accordingly, the defibrillator paddle 200 may comprise a locking or resistive mechanism for preventing or resisting movement of the second electrode plate 220.

Particularly, the second electrode plate 220 may comprise a biased plunger 410 configured to, when biased, prevent or resist movement of the second electrode plate 220. In particular, the biased plunger 410 may prevent or resist movement of the second electrode plate 220 at least when the defibrillator paddle 200 is in the first arrangement 201 or the second arrangement 202.

In the present embodiment, the biased plunger 410 may prevent or resist movement of the second electrode plate 220 by acting on the electrode plate holder 230. Accordingly, the biased plunger 410 may prevent or resist movement of the second electrode plate 220 along the axis A₁.

The biased plunger 410 may comprise a biasing element (e.g., a spring) that acts on (i.e., biases) a resistive element of the biased plunger 410. Particularly, when biased by the biasing element, the resistive element may act on the electrode plate holder 230, thereby producing a resistive force (e.g., friction) that resists movement of the second electrode plate 220.

In some examples, the second electrode plate 220 may only be moved (or at least become easier to move) when the biased plunger 410 is unbiased. Furthermore, it may be the case that the biased plunger 410 is automatically biased, i.e., in a 'normal' state, the biased plunger is biased. Accordingly, in order to move the second electrode plate 220, the biased plunger 420 may need to be actively (i.e., purposefully) unbiased, e.g., through something or someone acting on the biased plunger 420.

For example, the biased plunger 420 may become unbiased through application of a force to the biased plunger 420 in a direction opposite to the direction of biasing. Said force may be applied to the biased plunger 420 by a user (e.g., through said user gripping the biased plunger 420) or by a mechanical and/or electronic mechanism of the defibrillator paddle 200. Furthermore, said mechanism may be initiated in response to an input from a user.

Following with the present embodiment, the electrode plate holder 230 may comprise a first locking hole 420 and a second locking hole 430 configured to work in conjunction with the biased plunger 410. Specifically, when aligned with a respective locking hole, and when biased, the biased plunger 410 may be configured to enter (i.e., be received by) the respective locking hole. More specifically, each locking hole may be configured to receive a portion of the biased plunger 410. Accordingly, when receiving (the portion of) the biased plunger 410, each locking hole may prevent (or at least resist) movement of the second electrode plate 220.

Each locking hole may be associated with a particular arrangement of the defibrillator paddle 200. More particularly, a position of each locking hole on the electrode plate holder 230 may be representative of a desired position of the second electrode plate 220 for the first arrangement 201 and the second arrangement 202, respectively.

Specifically, the first locking hole 420 may be configured to receive the biased plunger 410 when the defibrillator paddle 200 is in the first arrangement 201. In other words, when the second shock surface 225 is aligned with the first plane P₁, the biased plunger 410 may be aligned with the first locking hole 420 such that it can be received by the first locking hole 420. Thus, when the biased plunger 410 is biased, movement of the second electrode plate 220 may be prevented when in the first arrangement 201.

Additionally, the second locking hole 430 may be configured to receive the biased plunger 410 when the defibrillator paddle 200 is in the second arrangement 202. In other words, when the second shock surface 225 is aligned with the second plane P₂, the biased plunger 410 may be aligned with the second locking hole 430 such that it can be received by the second locking hole 430. Thus, when the biased plunger 410 is biased, movement of the second electrode plate 220 may be prevented when in the second arrangement 202.

The electrode plate holder may further comprise a first microswitch and a second microswitch (not visible in Fig. 4). Specifically, the first microswitch may be arranged within the first locking hole 420 and the second microswitch may be arranged within the second locking hole 430.

Each microswitch may be activated through an applied force. In other words, each microswitch may be activated when acted on by another object. Particularly, the first microswitch may be activated (i.e., acted on) by the biased plunger 410 when received by the first locking hole 420. Additionally, the second microswitch may be activated by the biased plunger 410 when received by the second locking hole 430.

When activated/acted on, each microswitch may be configured to provide an indication or signal (such as a user-perceptible indication and/or an electrical signal) indicative of a particular arrangement of the defibrillator paddle 200. Specifically, when activated, the first microswitch may be configured to provide an indication of the defibrillator paddle 200 being in the first arrangement 201. This may particularly be the case when the first locking hole 420 is configured to only receive the biased plunger 410 in the first arrangement 201. Additionally, when activated, the second microswitch may be configured to provide an indication of the defibrillator paddle 200 being in the second arrangement 202. This may particularly be the case when the second locking hole 430 is configured to only receive the biased plunger 410 in the second arrangement 202.

The indication provided by each microswitch may be presented to a user of the defibrillator paddle 200 through a user-perceptible indication, e.g., a visual indication such as a light, and/or an audio indication. From the user-perceptible indication, the user may know which arrangement the defibrillator paddle 200 is in. Furthermore, the user-perceptible indication may confirm to the user when the defibrillator paddle 200 is in a desired arrangement, i.e., when the second electrode plate 220 is correctly positioned for a desired arrangement.

Alternatively, or additionally, the indication may be provided to a processing system of a defibrillator (e.g., the processing system 520 of Fig. 5). Accordingly, the processing system may modify one or more parameters of the defibrillator responsive to the received indication.

Fig. 5 schematically shows a proposed defibrillator 500. Particularly, the defibrillator 500 comprises at least one (though preferably two) defibrillator paddle(s) 200, and an electrical shock system 510 configured to provide electrical power to the defibrillator paddle(s) 200.

To provide more context, when the electrical shock system 510 (or the defibrillator 500 as a whole) is in an 'ON' state, i.e., receiving electrical power from a mains supply or internal battery, the electrical shock system 510 may store electrical energy, e.g., in a capacitor. Subsequently, following an activation indicator (e.g., a user input), the electrical shock system 510 may release said electrical energy and provide it to the defibrillator paddle(s) 200. Particularly, said activation indicator may be provided, e.g., by a user, following the defibrillator paddle(s) being ready for defibrillation, i.e., correctly positioned on a subject.

The defibrillator 500 further comprises a processing system 520 configured to control the electrical shock system 510. For example, the processing system 520 may control the provision of electrical energy/power from the electrical shock system 510 to the defibrillator paddle(s) 200. More specifically, the activation indicator may be provided to the processing system 520, where, responsive to the activation indicator, the processing system 520 may be configured to control the electrical shock system 510 to release the electrical energy.

The processing system 520 may also control one or more parameters of the released electrical energy/power/shock through controlling the electrical shock system 510. Particularly, the processing system 520 may control an output energy of the electrical shock system 510, i.e., the amount of energy provided to the defibrillator paddle(s) 200 by the electrical shock system 510. Said control of the output energy may be responsive to a user input, e.g., a user-specified amount as input by a user using a user interface of the defibrillator 500 (not visible in Fig. 5).

Preferably, the processing system 520 may (also) control the output energy responsive to an indication of the defibrillator paddle(s) 200 being in either the first arrangement or the second arrangement.

As previously disclosed, the first and second arrangements may correspond to preferred arrangements of the defibrillator paddle(s) 200 for defibrillating adult and child subjects, respectively. Furthermore, it is recognized that adult and child subjects may require different defibrillation energies for safe and effective defibrillation. Specifically, child subjects may require a defibrillation energy between 10-100 J, whereas adult subjects may require a defibrillation energy around 150 J. Accordingly, the defibrillation energy should preferably be changed dependent on the subject, which may automatically be adjusted from the arrangement of the defibrillator paddle(s) 200.

Responsive to an indication of the defibrillator paddle(s) 200 being in the first arrangement, the processing system 520 may control the output energy of the electrical shock system 510 to be approximately 150 J. Additionally, responsive to an indication of the defibrillator paddle(s) 200 being in the second arrangement, the processing system 520 may control the output energy of the electrical shock system 510 to be between 10-100 J.

The indication provided to the processing system 520 may be an electrical signal generated by a microswitch of the defibrillator paddle(s) 200. Particularly, when in the first arrangement, the microswitch may generate an electrical signal indicative of the defibrillator paddle(s) 200 being in the first arrangement. Additionally, when in the second arrangement, the microswitch may generate an electrical signal indicative of the defibrillator paddle(s) 200 being in the second arrangement.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A defibrillator paddle (200) comprising:
a first electrode plate (210) comprising a first shock surface (215); and
a second electrode plate (220) comprising a second shock surface (225), wherein the second electrode plate is configured to be moveable with respect to the first electrode plate;
wherein:
in a first arrangement (201) of the defibrillator paddle, the first shock surface and the second shock surface both lie in a first plane (P₁) and face a same direction; and
in a second arrangement (202) of the defibrillator paddle, the first shock surface lies in the first plane and the second shock surface lies in a second plane (Pz) different to the first plane;
wherein, in the first arrangement and the second arrangement of the defibrillator paddle, the first shock surface and the second shock surface are exposed.

2. The defibrillator paddle of claim 1, wherein:
the defibrillator paddle is configured to receive electrical power from an electrical shock system (510), wherein said electrical power is provided to at least the first electrode plate; and
in the first arrangement of the defibrillator paddle, the second electrode plate is configured to come into electrical contact with the first electrode plate.

3. The defibrillator paddle of claim 2, wherein said electrical contact is made via a plurality of resilient protrusions (310) arranged on the second electrode plate, wherein, in the first arrangement, the plurality of resilient protrusions are configured to come into contact with the first electrode plate.

4. The defibrillator paddle of any one of claims 2 or 3, wherein, in the second arrangement, the second electrode plate is electrically isolated from the first electrode plate.

5. The defibrillator paddle of any one of claims 1 to 4, wherein the second plane is parallel to the first plane.

6. The defibrillator paddle of any one of claims 1 to 5, further comprising an electrode plate holder (230) coupled to the first electrode plate and the second electrode plate, wherein:
the first electrode plate is rigidly connected to the electrode plate holder; and
the second electrode plate is configured to be moveable along an axis (A₁) of the electrode plate holder, wherein said axis makes a non-zero angle with respect to the first plane and the second plane.

7. The defibrillator paddle of claim 6, wherein the axis of the electrode plate holder is perpendicular to the first plane and the second plane.

8. The defibrillator paddle of any one of claims 6 or 7, wherein the first arrangement and the second arrangement of the defibrillator paddle correspond to extreme positions of the second electrode plate along the axis of the electrode plate holder.

9. The defibrillator paddle of any one of claims 6 to 8, wherein the second electrode plate comprises a biased plunger (410), wherein the biased plunger is configured to, when biased, prevent movement of the second electrode plate along the axis of the electrode plate holder at least when the defibrillator paddle is in the first arrangement and the second arrangement.

10. The defibrillator paddle of claim 9, wherein the electrode plate holder comprises:
a first locking hole (420) configured to receive the biased plunger in the first arrangement; and
a second locking hole (430) configured to receive the biased plunger in the second arrangement.

11. The defibrillator paddle of claim 10, wherein the electrode plate holder comprises a first microswitch and a second microswitch, wherein:
when received by the first locking hole, the biased plunger acts on the first microswitch; and
when received by the second locking hole, the biased plunger acts on the second microswitch;
wherein, when acted on by the biased plunger:
the first microswitch is configured to provide an indication of the defibrillator paddle being in the first arrangement; and
the second microswitch is configured to provide an indication of the defibrillator paddle being in the second arrangement.

12. The defibrillator paddle of any one of claims 1 to 11, wherein the second electrode plate comprises an aperture having bounds that surround the first electrode plate when the defibrillator paddle is in the first arrangement.

13. The defibrillator paddle of any one of claims 1 to 12, wherein an area of the second shock surface is greater than or equal to an area of the first shock surface.

14. A defibrillator (500) comprising:
an electrical shock system (510);
a processing system (520) configured to control the electrical shock system; and
at least one defibrillator paddle according to any one of claims 1 to 13, wherein the at least one defibrillator paddle is configured to receive electrical power from the electrical shock system.

15. The defibrillator of claim 14, wherein the processing system is configured to control at least an output energy of the electrical shock system responsive to an indication of the at least one defibrillator paddle being in either the first arrangement or the second arrangement.
